# EUROPEAN PATENT APPLICATION

(11) **EP 3 865 225 A1**
(43) Date of publication of application: **18.08.2021**
(21) Application number: 18826402.2
(22) Date of filing: 10.10.2018
(51) Int. Cl.: B09B 3/00, A61L 11/00

(54) **SYSTEM FOR CONTINUOUSLY TREATING SOLID WASTE AND ASSOCIATED PROCESS**

(71) Applicant: ECOHISPANICA I MAS D MEDIOAMBIENTAL, S.L., 28522 Rivas-Vaciamadrid (ES)
(72) Inventor: PACHECO, Jesús, 28522 Rivas-Vaciamadrid (ES); SOLER, Julián Alberto, 28522 Rivas-Vaciamadrid (ES)
(74) Representative: Pons
(86) International application number: PCT/ES2018/070658
(87) International publication number: WO 2020/074753

(57) **Abstract**

The present invention relates to a system for continuously treating solid waste and an associated process based on steam injection at a temperature greater than 100°C and at a pressure greater than the atmospheric pressure, which allow a high productivity to be achieved, require a smaller volume of the pressure vessel needed to carry out the solid waste treatment, and reduce the amount of steam needed to carry out said treatment.

## Description

### OBJECT OF THE INVENTION

The object of the present invention is a system for continuously treating solid waste and an associated process thereof based on steam injection at a temperature greater than 100°C and at a pressure greater than the atmospheric pressure.

Due to the special configuration thereof, the system and process for continuously treating solid waste allow a high productivity to be achieved, require a smaller volume of the pressure vessel in order to carry out the solid waste treatment and reduce the steam needed to carry out said treatment.

### BACKGROUND OF THE INVENTION

Waste treatment devices and processes are known in the state of the art.

Known in the prior art is the European patent with publication number EP2519362A1 by the same applicant of the present application, relating to a device and procedure for the continuous treatment of waste which is actually a semi-continuous process comprising the following steps:
- placing the material to be treated in a pressure tank;
- causing the material to move through the tank in a substantially continuous and uninterrupted manner with a first helical blade through a first inner flow path in the tank in a first axial direction and with a second helical blade through a second outer flow path in a second axial direction opposite to the first axial direction, the first and second flow paths being generally concentric, and
- extracting the material from the pressure tank.

Also known is patent US7303160B2 which relates to a system and method for processing waste on a continuous basis comprising an infeed assembly, a vessel held at a non-ambient pressure and/or temperature, and an outfeed assembly. The infeed assembly includes: a sleeve where waste is delivered; a gate construction that moves between an open and a closed position; and a ram that moves within the sleeve to compress waste when the gate construction is in the closed position and pushes the compressed waste into the vessel. The processed material moves from the vessel to the outfeed assembly, which includes a compaction chamber, a piston that moves within the compaction chamber, and a cutter assembly. Waste is compacted in the compaction chamber by the ram, cut by the cutter assembly, and delivered as a compacted block to an environment having an ambient pressure and/or temperature.

The foregoing systems and methods have the following drawbacks:
- although they relate to continuous treatments, they actually do not maintain a continuous flow of solid waste to the pressure equipment, since in said systems, the feeding and extraction of solid waste is done in a discreet way by means of inlet and outlet devices designed to respectively feed or extract batches of material at a frequency determined by the number of operations and the time of each one;
- the heating times of the solid waste are long since the mechanical devices used for mixing steam have a low efficiency, such as auger mixers, drum turners or blades;
- they require free space in the pressure vessel to use mechanical elements for mixing and transporting the solid waste through the inside thereof;
- the heating times are long due to the fact that they use mechanical elements with low efficiency for mixing the solid waste with the steam and the presence of trapped air in the incoming solid waste worsens the heat transfer;
- they require a high number of devices and pressure vessels to be able to treat batches of solid waste;
- the pressure vessels have expensive mechanical devices for transporting and heating the solid waste inside the same;
- automation and control of batch processing are highly complex;
- they require depressurisation of the inlet device every time a new batch of solid waste is fed to the pressure vessel.
- they need high volume pressure vessels for the same production capacity;
- the generation of a discontinuous flow of flash steam due to the fact that the discharge of the treated solid waste is not continuous, which makes energy recovery of said waste steam current difficult.

The system for continuously treating solid waste and the associated process thereof of the present invention solve all of the previously mentioned drawbacks.

### DESCRIPTION OF THE INVENTION

A first aspect of the present invention describes a system for continuously treating solid waste comprising:
- a pumping device configured to continuously pump and compact a solid waste flow, wherein the pumping device comprises an inlet configured to carry out the suction of the solid waste flow at a first pressure comprised in the range [0; 1] barg, in other words, at a relative pressure with respect to the atmospheric pressure, and an outlet configured to pump the solid waste flow at a second pressure that is greater than the first pressure;
- a pressurised tubular vessel configured to maintain the pressure of the solid waste flow at the second pressure;
- at least one steam injection device for injecting steam in the solid waste, configured to heat and break up the solid waste inside the pressurised tubular vessel; and
- an extraction device configured for extracting the treated solid waste from the pressurised tubular vessel and for reducing the pressure of the flow of the treated solid waste in a controlled manner from the second pressure to the first pressure.

The impact of the injected steam by the at least one steam injection device on the solid waste has a physical effect of erosion and breakup, which allows for the fast diffusion of the same towards the centre of the block that forms said solid waste by circulating through the pressurised tubular vessel. In this way a practically instantaneous heating of the solid waste flow that passes through said pressurised tubular vessel is achieved.

Optionally, the system comprises control means configured for controlling the treatment temperature of the solid waste, regulating the steam injection by means of the at least one steam injection device for injecting steam in the solid waste. This way the temperature of the solid waste downstream from the at least one steam injection device is controlled.

Optionally, the system comprises control means configured for controlling the pressure of the treated solid waste flow downstream from the extraction device, regulating the extraction speed of said treated solid waste, which is the treatment pressure.

Optionally, the system comprises control means configured for controlling the treated solid waste flow introduced in the pressurised tubular vessel, regulating the pumping speed of said solid waste by means of the pumping device.

The residence time of the solid waste circulating inside the pressurised tubular vessel will depend on the type of solid waste as well as the pressure and temperature conditions chosen for the treatment thereof.

Optionally, the system further comprises a flash chamber arranged after the extraction device, wherein the flash chamber comprises an assembly of breakers configured for crumbling the treated solid waste.

Optionally, the system further comprises a hopper configured to receive the solid waste prior to the passage thereof through the pumping device.

Also, optionally, the system comprises a preheating and degassing device configured to preheat the solid waste flow and eliminate air trapped between the particles of said solid waste prior to the passage thereof through the pumping device.

The preheating and degassing device uses waste steam from coming from different areas of the system, such as flash steam after depressurisation of the treated solid waste at the outlet of the extraction device.

This way the system minimises energy consumption due to the preheating of the solid waste that is done in the preheating and degassing device by means of a waste stream flow, in turn reducing the treatment time of the solid waste by eliminating the air trapped between the solid waste particles at the entrance of the system which improves heat transfer.

Furthermore, the pressure drop in the pumping device is reduced and the steam diffusion in the pressurised tubular vessel is improved due to the increase in plasticity as a result of the preheating of the incoming solid waste.

In turn, the preheating and degassing device can incorporate a device for the inlet of water and/or condensates recovered from the process configured to adjust the moisture of the solid waste prior to the passage thereof through the pumping device, putting the water and/or condensates in contact with the solid waste in the preheating and degassing device. Depending on the type of solid waste, said increase in moisture by means of the inlet of water and/or condensates through the device for the inlet of water and/or condensates and/or by means of a device for the inlet of flash steam, could be necessary in order to increase the plasticity thereof and improve the treatment of the same in the line by means of the steam injection device. This way, the system comprises means for regulating the inlet of water and/or condensates by means of the device for the inlet of water and/or condensates and/or by means of the device for the inlet of flash steam into the preheating and degassing device.

A second aspect of the present invention describes a process for continuously treating solid waste comprising the following stages:
- a pumping stage wherein the pumping of a solid waste flow is carried out in a continuous and compact way from a first pressure comprised in the range [0; 1] barg, in other words, at a relative pressure with respect to the atmospheric pressure, to a second pressure that is greater than the first pressure;
- a stage of maintaining the pressure of the solid waste flow in a confined way at the second pressure;
- a stage of injecting steam in the solid waste, wherein the heating and breakup of the solid waste is carried out in a confined way; and
- a stage of extracting the treated solid waste, wherein the reduction of the pressure of the flow of the treated solid waste is carried out in a controlled manner from the second pressure to the first pressure.

Optionally, the process comprises a stage of controlling the temperature of the solid waste by means of which the temperature of the solid waste is controlled after the stage of injecting steam in the solid waste.

Optionally, the process comprises a stage of controlling the solid waste flow before the stage of injecting steam in the solid waste.

Optionally, the process comprises a stage of controlling the pressure of the treatment of the solid waste before the stage of extracting said treated waste.

Optionally, the process further comprises a flash stage after the extraction stage wherein the crumbling of the treated solid waste is carried out.

Optionally, the process further comprises a stage of preheating and degassing the solid waste flow before the pumping stage. Based on the type of solid waste, said stage can include a sub-stage of the injection of water and/or condensates recovered from other parts of the process in order to increase the moisture and improve the subsequent treatment in the line of the solid waste in the steam injection stage.

This way, the system for continuously treating solid waste and the associated process thereof have the following advantages:
- high productivity associated with maintaining a continuous pressurised flow of solid waste to the tubular vessel and performing a treatment in the line by steam injection;
- lower volume of the pressure vessel needed to carry out the solid waste treatment associated with the complete filling of said vessel and with heating in the line by steam injection;
- simplicity of the process associated with maintaining a continuous pressurised flow of solid waste to the tubular vessel and performing a treatment in the line by steam injection;
- less steam consumption needed for treating the waste due to three factors:
   - continuous feeding of solid waste to the pressure vessel without a loss of steam;
   - less heat loss to the exterior by requiring a smaller size of the pressure vessel;
   - continuous discharge of the treated solid waste, which allows the flash steam to be continuously recovered and used for degassing and preheating the incoming solid waste.

### DESCRIPTION OF THE DRAWINGS

As a complement to the description provided herein and for the purpose of helping to make the characteristics of the invention more readily understandable, in accordance with a preferred practical embodiment thereof, said description is accompanied by a set of drawings constituting an integral part of the same, which by way of illustration and not limitation represent the following:

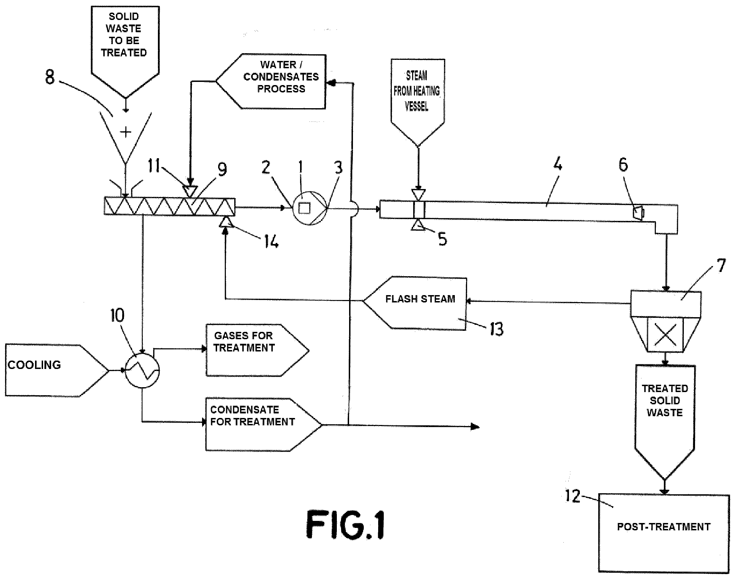

Figure 1 shows a diagram of the system for continuously treating solid waste and the associated process thereof of the present invention.

### PREFERRED EMBODIMENT OF THE INVENTION

The system and process for continuously treating solid waste of the present invention is described in a detailed way below.

The system for continuously treating solid waste comprises:
- a pumping device (1), preferably a positive displacement pump, configured to continuously pump and compact a solid waste flow, wherein the pumping device (1) comprises an inlet (2) configured to carry out the suction of the solid waste flow at a first pressure comprised in the range [0; 1] barg, in other words, at a relative pressure with respect to the atmospheric pressure, preferably comprised in the range of [0; 0.5] barg, and an outlet (3) configured to pump the solid waste flow at a second pressure that is greater than the first pressure comprised in the range [1; 20] barg, preferably comprised in the range [2; 10] barg;
- a pressurised tubular vessel (4), preferably with a circular cross section, configured to maintain the pressure of the solid waste flow at the second pressure;
- at least one steam injection device (5) for injecting steam in the solid waste, configured to heat and break up of the solid waste inside the pressurised tubular vessel (4); wherein the at least one steam injection device (5) is inserted along the pressurised tubular vessel (4); and
an extraction device (6), preferably a positive displacement device, configured for extracting the treated solid waste from the pressurised tubular vessel (4) and for reducing the pressure of the flow of the treated solid waste in a controlled manner, preferably with each positive displacement, from the first pressure to the second pressure. There is a residence time of the treated solid waste in the pressurised tubular vessel (4) that depends on the volume of the section of the tubular vessel comprised between the steam injection device (5) and the extraction device (6), as well as on the waste flow pumped by the pumping device (1).

The system further comprises a flash chamber (7) arranged after the extraction device (6), wherein the flash chamber (7) comprises an assembly of breakers configured for crumbling the treated solid waste.

The system further comprises a hopper (8) configured to receive the solid waste prior to the passage thereof through the pumping device (1).

The system comprises a preheating and degassing device (9) configured to preheat the solid waste flow and eliminate air trapped between the particles of said solid waste prior to the passage thereof through the pumping device (1), wherein the preheating and degassing device (9) is a solid-steam counterflow heat exchanger.

The system comprises a condensation device (10) to which the waste steam that has not condensed in the preheating and degassing device (9) is sent.

The preheating and degassing device (9) further comprises nozzles (11) for the injection of water and/or condensates recovered from the process for moistening the solid waste and improving the subsequent treatment in the line thereof by means of the steam injection devices.

The system further comprises one or several post-treatment devices (12) which, based on the quality of the final material to be obtained, could be a dryer, a sieve and a densimetric separator, configured to carry out the drying and/or separation by particle size and/or densimetric separation of the treated solid waste.

The process for continuously treating solid waste comprises the following stages:
- a pumping stage wherein the pumping of a solid waste flow is carried out in a continuous and compact way, from a first pressure comprised in the range [0; 1] barg, in other words, at a relative pressure with respect to the atmospheric pressure, preferably comprised in the range [0; 0.5] barg, to a second pressure that is greater than the first pressure, comprised in the range [1; 20] barg, preferably comprised in the range [2; 10] barg;
- a stage of maintaining the pressure of the solid waste flow in a confined manner at the second pressure;
- a stage of injecting steam in the solid waste, preferably at speeds over 20 m/s, wherein the heating and breakup of the solid waste is carried out in a confined way; and
- an extraction stage of the treated solid waste wherein the reduction of the pressure of the flow of the treated solid waste is carried out in a controlled manner from the second pressure to the first pressure. Preferably, the steam injection stage is carried out in a uniformly distributed fashion on the entire perimeter of the solid waste and/or on several sections of the same.

Said stage of injecting steam in the solid waste is a steam injection stage at a temperature above 100°C.

Due to the special configuration thereof, the system and process for continuously treating solid waste allow a high productivity to be achieved, require a smaller volume of the pressure vessel in order to carry out the solid waste treatment and reduce the steam consumption needed to carry out said treatment.

The process further comprises a flash stage after the extraction stage wherein the crumbling of the treated solid waste is carried out.

The process further comprises a stage of preheating and degassing the solid waste flow prior to the pumping stage. Based on the type of solid waste, said stage can include a sub-stage of injection of water or condensates recovered from the process in order to increase the moisture and improve the subsequent treatment in the line of the solid waste in the steam injection stage.

The process further comprises a stage of condensing the excess waste steam after the preheating and degassing stage.

The process further comprises one or several post-treatment stages, wherein the drying and/or separation by particle size and/or densimetric separation of the treated solid waste is carried out.

### EXAMPLE

Several tests of continuously treating solid waste were carried out. In several of them the organic fraction of municipal solid waste (OFMSW) with a particle size of less than 80 mm mesh size was used as waste.

In the previous stage of preheating, degassing and moisture adjustment, the solid waste is conditioned by injecting steam and condensates recovered from the process to bring it to a temperature of 80°C, eliminating the gasses trapped between the particles thereof and adjusting the moisture thereof from initial approximately 48% up to the complete saturation thereof corresponding to 100% of moisture.

A solid waste flow of 15 t/h is adjusted at the beginning of the process which, after passing through the preheater and degasser, is sucked by the positive displacement pump at a pressure of approximately 0.2 barg and pumped at a second selected working pressure of 5 barg.

After entering into the pressurised tubular vessel, the injection of the steam from the heating vessel takes place, which breaks up and heats the solid waste to the temperature of the saturated steam at the selected working pressure. To complete the treatment, said working temperature is maintained during a residence time of the solid waste in the pressurised tubular vessel for 10 minutes.

Lastly, the treated solid waste is depressurised in a controlled way with the resulting recovery of the steam (13) produced by the flash of the material.

## Claims

1. A system for continuously treating solid waste, **characterised in that** it comprises:
• a pumping device (1) configured to continuously pump and compact a solid waste flow, wherein the pumping device (1) comprises an inlet (2) configured to carry out the suction of the solid waste flow at a first pressure comprised in the range [0; 1] barg, in other words, at a relative pressure with respect to the atmospheric pressure, and an outlet (3) configured to pump the solid waste flow at a second pressure that is greater than the first pressure;
• a pressurised tubular vessel (4) configured to maintain the pressure of the solid waste flow at the second pressure;
• at least one steam injection device (5) for injecting steam in the solid waste, configured to heat and break up the solid waste inside the pressurised tubular vessel (4);
• an extraction device (6) configured for extracting the treated solid waste from the pressurised tubular vessel (4) and for reducing the pressure of the flow of the treated solid waste in a controlled manner from the first pressure to the second pressure.

2. The system for continuously treating solid waste according to claim 1, **characterised in that** it further comprises a flash chamber (7) arranged after the extraction device (6), wherein the flash chamber (7) comprises an assembly of breakers configured for crumbling the treated solid waste.

3. The system for continuously treating solid waste according to any of the preceding claims, **characterised in that** it further comprises a hopper (8) configured to receive the solid waste prior to the passage thereof through the pumping device (1).

4. The system for continuously treating solid waste according to any of the preceding claims, **characterised in that** it further comprises a preheating and degassing device (9) configured to preheat the solid waste flow and eliminate the trapped air between the particles of said solid waste prior to the passage thereof through the pumping device (1).

5. The system for continuously treating solid waste according to claims 2 and 4, **characterised in that** the preheating and degassing device (9) comprises a device for the inlet of water and/or condensates (11) recovered from the process and/or a device for the inlet (14) of flash steam into the preheating and degassing device configured for adjusting the moisture of the solid waste prior to the passage thereof through the pumping device.

6. The system for continuously treating solid waste according to claim 5, **characterised in that** it comprises means for regulating the water inlet and/or condensates by means of the device for the inlet of water and/or condensates and/or the device for the inlet of flash steam into the preheating and degassing device.

7. The system for continuously treating solid waste according to any of claims 4 to 6, **characterised in that** it further comprises a condensation device (10) to which the waste steam that has not condensed in the preheating and degassing device (9) is sent.

8. The system for continuously treating solid waste according to any of the preceding claims, **characterised in that** it further comprises one or several post-treatment devices (12) configured to carry out the drying and/or separation by particle size and/or densimetric separation of treated solid waste.

9. The system for continuously treating solid waste according to any of the preceding claims, **characterised in that** the pumping device (1) is a positive displacement device.

10. The system for continuously treating solid waste according to any of the preceding claims, **characterised in that** the pressurised tubular vessel (4) has a circular cross section.

11. The system for continuously treating solid waste according to claim 1, **characterised in that** the at least one steam injection device (5) for injecting steam in the solid waste is inserted along the pressurised tubular vessel (4).

12. The system for continuously treating solid waste according to any of the preceding claims, **characterised in that** the extraction device (6) is a positive displacement device, configured for extracting the treated solid waste from the pressurised tubular vessel (4) and for reducing the pressure of the flow of the treated solid waste in a controlled manner, preferably with each positive displacement, from the first pressure to the second pressure.

13. The system for continuously treating solid waste according to any of the preceding claims, **characterised in that** the preheating and degassing device (9) is a solid-steam counterflow heat exchanger.

14. The system for continuously treating solid waste according to any of the preceding claims, **characterised in that** the outlet (3) of the pumping device (1) is configured for pumping the solid waste flow at the second pressure, greater than the first pressure, comprised in the range [1; 20] barg.

15. The system for continuously treating solid waste according to claim 14, **characterised in that** at the inlet (2) of the pumping device (1) the suction of the solid waste flow is carried out at a first pressure comprised in the range [0; 0.5] barg, and at the outlet (3) the pumping of the solid waste flow is carried out at the second pressure, comprised in the range [2; 10] barg.

16. The system for continuously treating solid waste according to any of the preceding claims, **characterised in that** it comprises control means configured for controlling the treatment temperature of the solid waste, regulating the steam injection by means of the at least one steam injection device (5) for injecting steam in the solid waste.

17. The system for continuously treating solid waste according to any of the preceding claims, **characterised in that** it comprises control means configured for controlling the pressure of the treated solid waste flow downstream from the extraction device (6) regulating the extraction speed of said treated solid waste.

18. The system for continuously treating solid waste according to any of the preceding claims, **characterised in that** it comprises control means configured for controlling the flow of the solid waste that is introduced in the pressurised tubular vessel (4) regulating the pumping speed of said solid waste by means of the pumping device (1).

19. A process for continuously treating solid waste, **characterised in that** it comprises the following stages:
• a pumping stage configured for carrying out the pumping of a solid waste flow in a continuous and compact way, from a first pressure comprised in the range [0; 1] barg, in other words, at a relative pressure with respect to the atmospheric pressure, to a second pressure that is greater than the first pressure;
• a stage of maintaining the pressure of the solid waste flow in a confined manner at the second pressure;
• a stage of injecting steam in the solid waste, wherein the heating and breakup of the solid waste is carried out in a confined way; and
• a stage of extracting the treated solid waste, wherein the reduction of the pressure of the flow of the treated solid waste is carried out in a controlled manner from the second pressure to the first pressure.

20. The process for continuously treating solid waste according to claim 16, **characterised in that** the stage injecting steam in the solid waste is carried out at speeds greater than 20 m/s.

21. The process for continuously treating solid waste according to any of the claims 19 or 20, **characterised in that** the stage of injecting steam in the solid waste is carried out in a uniformly distributed manner throughout the entire perimeter of the solid waste and/or in several sections of the same.

22. The process for continuously treating solid waste according to any of the claims 19 to 21, **characterised in that** the stage of injecting steam in the solid waste is a stage of injecting steam at a temperature greater than 100°C.

23. The process for continuously treating solid waste according to any of claims 19 to 22, **characterised in that** it further comprises a flash stage after the extraction stage wherein the crumbling of the treated solid waste is carried out.

24. The process for continuously treating solid waste according to any of the claims 19 to 21, **characterised in that** it further comprises a stage of preheating and degassing the solid waste flow prior to the pumping stage.

25. The process for continuously treating solid waste according to claim 24, **characterised in that** the stage of preheating and degassing the solid waste flow comprises a sub-stage of the injection of water and/or condensates recovered from the process.

26. The process for continuously treating solid waste according to any of the claims 19 to 25, **characterised in that** it further comprises one or several post-treatment stages wherein the drying and/or separation by particle size and/or densimetric separation of treated solid waste is carried out.

27. The process for continuously treating solid waste according to any of the claims 19 to 26, **characterised in that** the second pressure of the pumping stage is comprised in the range [1; 20] barg.

28. The process for continuously treating solid waste according to claim 27, **characterised in that** the first pressure of the pumping stage is comprised in the range [0; 0.5] barg and the second pressure is comprised in the range [2; 10] barg.

29. The process for continuously treating solid waste according to any of the claims 19 to 28, **characterised in that** it comprises a stage of controlling the temperature of the solid waste after the stage of injecting steam in the solid waste.

30. The process for continuously treating solid waste according to any of the claims 19 to 29, **characterised in that** it comprises a stage of controlling the solid waste flow before the stage of injecting steam in the solid waste.

31. The process for continuously treating solid waste according to any of the claims 19 to 30, **characterised in that** it comprises a stage of controlling the treatment pressure of the solid waste before the extraction of said treated waste.
